(19) Europäisches Patentamt
European Patent Office
Office européen des brevets

(11) Veröffentlichungsnummer: **0 172 509**
**A2**

(12) # EUROPÄISCHE PATENTANMELDUNG

(21) Anmeldenummer: 85110052.9

(22) Anmeldetag: 09.08.85

(51) Int. Cl.⁴: C 07 D 413/04
C 07 D 493/04, C 07 B 57/00
A 61 K 31/44, C 07 D 271/08
C 07 C 69/72, C 07 C 119/06
//(C07D493/04, 307:00, 307:00)

(30) Priorität: 24.08.84 DE 3431152

(43) Veröffentlichungstag der Anmeldung: 26.02.86 Patentblatt 86/9

(84) Benannte Vertragsstaaten: AT BE CH DE FR GB IT LI NL SE

(71) Anmelder: CASSELLA Aktiengesellschaft
Hanauer Landstrasse 526
D-6000 Frankfurt am Main 61(DE)

(72) Erfinder: Schönafinger, Karl, Dr.
Holunderweg 8
D-8755 Alzenau(DE)

(72) Erfinder: Bohn, Helmut, Dr.
Kranzbergring 11
D-6369 Schöneck(DE)

(72) Erfinder: Martorana, Piero A. Dr.
Kaiser-Friedrich-Promenade 108
D-6380 Bad Homburg(DE)

(72) Erfinder: Nitz, Rolf-Eberhard, Dr.
Heinrich-Bingemer-Weg 64
D-6000 Frankfurt am Main 60(DE)

(74) Vertreter: Urbach, Hans-Georg, Dr. et al.
Hanauer Landstrasse 526
D-6000 Frankfurt am Main 61(DE)

(54) **Verfahren zur Herstellung optisch aktiver, substituierter 1,4-Dihydropyridine und ihre Verwendung als Arzneimittel.**

(57) Optisch aktive, substituierte 1,4-Dihydropyridine der Formel I

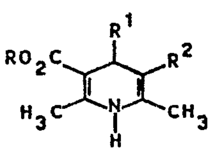

(I)

worin
R z.B. $R^3$,
$R^1$ z.B. ein gegebenenfalls substituiertes Phenyl, Pyridyl oder Thienyl,
$R^2$ den Rest eines 5-gliedrigen, gegebenenfalls substituierten Rings mit mindestens einer Doppelbindung und mindestens 2 Heteroatomen oder Heteroatomgruppierungen aus der Reihe O, N, NH, S,
$R^3$ z.B. Alkyl oder alkoxyalkyl
bedeuten, und ihre Säureadditionssalze besitzen wertvolle pharmakologische Eigenschaften.

0172509
Dr.Eu/Ll

# Verfahren zur Herstellung optisch aktiver, substituierter 1,4-Dihydropyridine und ihre Verwendung als Arzneimittel

Die Erfindung betrifft ein Verfahren zur Herstellung optisch aktiver, substituierter 1,4-Dihydropyridine der Formel I

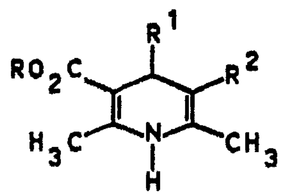

(I)

und ihrer Säureadditionssalze, wobei in der Formel I

R den Rest $R^3$ oder $R^4$,

$R^1$ Pyridyl oder Thienyl, wobei der Pyridyl- oder Thienyl-Rest gegebenenfalls 1 oder 2 gleiche oder verschiedene Substituenten aus der Gruppe Alkyl mit 1 bis 4 C-Atomen, Alkoxy mit 1 bis 4 C-Atomen, Halogen, Trifluormethyl, Nitro, Cyano besitzt; Phenyl, das gegebenenfalls 1 oder 2 gleiche oder verschiedene Substituenten aus der Gruppe Halogen, Nitro, Cyano, Trifluormethyl, Alkyl mit 1 bis 4 C-Atomen, Alkoxy mit 1 bis 4 C-Atomen besitzt,

$R^2$ den Rest eines 5-gliedrigen Rings mit mindestens einer Doppelbindung und mindestens 2 Heteroatomen oder Heteroatomgruppierungen aus der Reihe O, N, NH, S, wobei der 5-gliedrige Ring gegebenenfalls 1 oder 2 gleiche oder verschiedene Substituenten aus der Gruppe Alkyl mit 1 bis 4 C-Atomen, Alkylthio mit 1 bis 4 C-Atomen, Aralkyl mit insgesamt 7 bis 9 C-Atomen, Alkoxyalkyl mit insgesamt 2 bis 5 C-Atomen, Cycloalkyl mit 5 oder 6 C-Atomen, Aminocarbonylmethylthio, Methoxy-carbonyl, Ethoxy-carbonyl, Phenyl besitzt,

$R^3$ Alkyl mit 1 bis 6 C-Atomen, Alkoxyalkyl mit 3 bis 8 C-Atomen, Dialkylaminoalkyl mit insgesamt 4 bis 9 C-Atomen, N-Aralkyl-N-alkyl-aminoalkyl mit insgesamt 10

bis 14 C-Atomen, Cycloalkyl mit 5 oder 6 C-Atomen

$R^4$ den Rest $(R)-CH(CH_3)CO_2R^5$, $(S)-CH(CH_3)CO_2R^5$ oder den Rest der Formel II

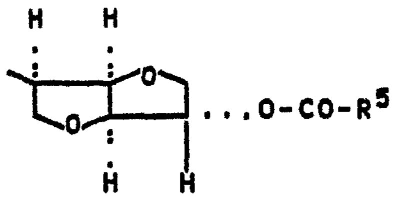 (II)

$R^5$ Alkyl mit 1 bis 6 C-Atomen

bedeuten.

Die Erfindung betrifft auch die optisch aktiven Verbindungen der Formel I sowie ihre Verwendung als Arzneimittel.

Die genannten Reste Alkyl und Alkoxy sind, auch wenn sie in Kombinationen miteinander oder in anderen Resten, wie z.B. Alkoxyalkyl, Aralkyl, Dialkylaminoalkyl, Alkoxycarbonyl oder als Substituenten für andere Reste vorkommen, geradkettig oder verzweigt. Sofern für sie oder für die sie enthaltenden Gruppierungen nicht bereits vorstehend Bereiche für ihre Kohlenstoffzahl angegeben sind, enthalten sie normalerweise 1 bis 4 C-Atome.

Die genannten Aralkylreste sind insbesondere Phenalkylreste, so z.B. Phenylpropyl, Phenylethyl oder Benzyl, von denen Phenylethyl und insbesondere Benzyl bevorzugt sind.

Halogen bedeutet in der Regel Chlor, Brom oder Fluor, vorzugsweise Chlor oder Brom, ganz besonders bevorzugt Chlor.

$R^1$ kann z.B. ein 2-, 3- oder 4-Pyridylrest oder ein 2- oder 3-Thienylrest sein, wobei diese Reste ein oder zwei gleiche oder verschiedene Substituenten besitzen können.

$R^1$ bedeutet vorzugsweise Phenyl, das gegebenenfalls ein oder zwei gleiche oder verschiedene Substituenten, vorzugsweise aus der Reihe Chlor, Brom, Fluor, Nitro, Cyano, Methyl, Methoxy, Trifluormethyl, besitzt. Beispiele für derartige für $R^1$ stehende Reste sind: Phenyl, 2-Chlorphenyl, 3-Chlorphenyl, 4-Chlorphenyl, 2,3-Dichlorphenyl, 2-Nitrophenyl, 3-Nitrophenyl, 3-Cyanophenyl, 3-Methoxyphenyl, 3-Trifluormethylphenyl, 2-Trifluormethylphenyl, o-Tolyl, m-Tolyl oder p-Tolyl.

Besonders bevorzugt bedeutet $R^1$ ein durch Cyan, Nitro oder Chlor monosubstituiertes oder durch Chlor disubstituiertes Phenyl, wobei die Substituenten vorzugsweise in 2- und/oder 3-Stellung des Phenylkerns vorhanden sind. Ganz besonders bevorzugt bedeutet $R^1$ 2-Nitrophenyl, 3-Nitrophenyl, 3-Cyanophenyl, 2-Chlorophenyl und 2,3-Dichlorophenyl.

$R^2$ kann z.B. ein Oxazolyl-, Thiazolyl-, Imidazolyl-, Triazolyl-, Oxadiazolyl-, Thiadiazolyl-Rest sein. Als Substituenten für die Reste $R^2$ kommen z.B. in Betracht: Methyl, Ethyl, n-Propyl, i-Propyl, n-Butyl, i-Butyl, tert.Butyl, Benzyl, Methylthio, i-Propylthio, Methoxymethyl, 2-Methoxyethyl, Aminocarbonylmethylthio, Methoxy-carbonyl, Ethoxy-carbonyl, Cyclopentyl, Cyclohexyl, Phenyl. Bei den für $R^2$ stehenden Resten von 5-gliedrigen Ringen sind solche bevorzugt, die zwei Stickstoffatome und ein Sauerstoffatom und zwei Doppelbindungen enthalten, wie z.B. 1,3,4-Oxadiazol-2-yl, 1,2,4-Oxadiazol-5-yl, 1,2,4-Oxadiazol-3-yl. Bevorzugte Substituenten sind: Methyl, Ethyl, i-Propyl, tert.Butyl, Benzyl, Methylthio, i-Propylthio, Aminocarbonylmethylthio, Methoxymethyl. Besonders bevorzugte Substituenten sind: Methyl, Ethyl

und Benzyl. Besonders bevorzugt für $R^2$ sind 1,3,4-Oxadiazol-2-yl, 5-Methyl-1,3,4-oxadiazol-2-yl, 5-Ethyl-1,3,4-oxadiazol-2-yl, 3-Methyl-1,2,4-oxadiazol-5-yl, 3-Ethyl-1,2,4-oxadiazol-5-yl, 3-Benzyl-1,2,4-oxadiazol-5-yl.

Bei dem für $R^3$ stehenden N-Aralkyl-N-alkyl-aminoalkyl-Rest sitzt die N-Aralkyl-N-alkyl-aminogruppe insbesondere an dem endständigen C-Atom des Alkylrestes, wie z.B.: 2-(N-Benzyl-N-methyl-amino)-ethyl, 2-(N-Phenethyl-N-methylamino)-ethyl, 2-(N-Benzyl-N-ethyl-amino)-ethyl.

Vorzugsweise bedeutet $R^3$ Alkyl mit 1 bis 5 C-Atomen, Alkoxyalkyl mit 1 bis 4 C-Atomen im Alkoxyteil und 2 bis 4 C-Atomen im Alkylteil, Dialkylaminoalkyl mit insgesamt 3 bis 6 C-Atomen, wobei jede der die Aminogruppe substituierenden Alkylgruppen 1 bis 3 C-Atome besitzen kann und wobei bei dem Alkoxyalkylrest die Alkoxygruppe und bei dem Dialkylaminoalkylrest die Dialkylaminogruppe, insbesondere an dem endständigen C-Atom des Alkylrestes, sitzen. Beispiele für derartige bevorzugte Reste $R^3$ sind: Methyl, Ethyl, n-Propyl, i-Propyl, n-Butyl, i-Butyl, sec.-Butyl, tert.-Butyl, Neopentyl, 2-Methoxy-ethyl, 2-i-Propoxy-ethyl, 2-n-Butoxy-ethyl, 3-Methoxy-n-propyl, 2-Dimethylamino-ethyl. Besonders bevorzugt bedeutet $R^3$ Methyl, n-Propyl, n-Butyl, i-Butyl, tert.Butyl und 2-i-Propoxy-ethyl. Ganz besonders bevorzugt bedeutet $R^3$ i-Propyl und 2-Methoxy-ethyl.

Der Rest $R^4$ steht für den Rest $-CH(CH_3)CO_2R^5$ in optisch aktiver (R)- oder (S)-Form, der sich von einem optisch aktiven (R)- bzw. (S)-Milchsäureester der Formel $HO-CH(CH_3)CO_2R^5$ ableitet. Der Rest $R^4$ steht darüber hinaus vorzugsweise für den Rest der Formel II

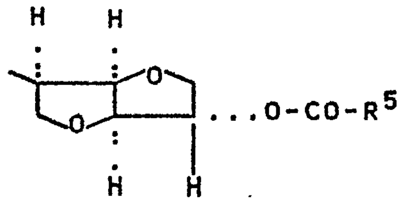

(II)

der sich von einem 2-O-($R^5$-carbonyl)-isosorbid der Formel IIa

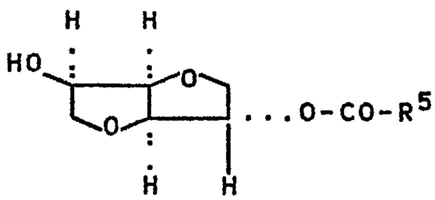

(IIa)

ableitet. Die Verbindung, die in der Formel IIa anstelle des $-CO-R^5$-Restes ein -H besitzt und die demnach die Formel IIb

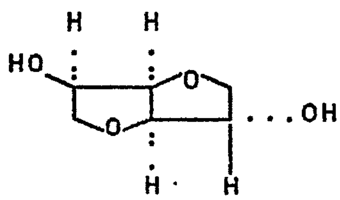

(IIb)

besitzt, wird z.B. als Isosorbid, 1,4 : 3,6-Dianhydro-D-glucitol oder 1,4 : 3,6-Dianhydro-D-sorbit bezeichnet. Im Rahmen der vorliegenden Erfindung wird der optisch aktive Rest der Formel II aus Vereinfachungsgründen als 2-O-($R^5$-carbonyl)-isosorbid-5-yl bezeichnet. Vorzugsweise steht $R^5$ für Alkyl mit 1 bis 4 C-Atomen, insbesondere für Methyl oder Ethyl. Der Rest $R^5$ in der Formel II bedeutet vorzugsweise Methyl, so daß für II der Rest 2-O-Acetyl-isosorbid-5-yl besonders bevorzugt ist.

Bevorzugte Verbindungen der Formel I sind solche, bei denen die Reste eine oder insbesondere mehrere der angegebenen bevorzugten Bedeutungen besitzen. Ganz besonders bevorzugte Verbindungen sind solche, bei denen die Reste eine oder insbesondere mehrere, vorzugsweise alle der angegebenen, besonders bevorzugten Bedeutungen besitzen.

Verbindungen der Formel I mit $R = R^4$ werden nachfolgend als Verbindungen der Formel Ia, und Verbindungen der Formel I mit $R = R^3$ werden nachfolgend als Verbindungen der Formel Ib bezeichnet:

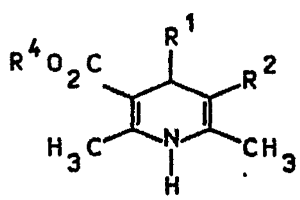

(Ia)

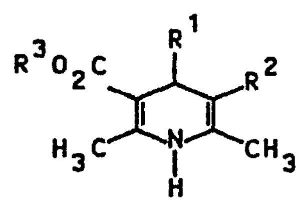

(Ib)

Optisch inaktive Verbindungen der Formel Ib sind in der europäischen Patentanmeldung mit der Anmeldungsnummer 83112562.0, Veröffentlichungsnummer 0116708, beschrieben. Es wurde nun gefunden, daß sich die optisch aktiven Verbindungen der Formel Ib in eleganter Weise dadurch herstellen lassen, daß eine optisch aktive Verbindung der Formel Ia mit einem Alkohol der Formel $R^3OH$ umgeestert wird:

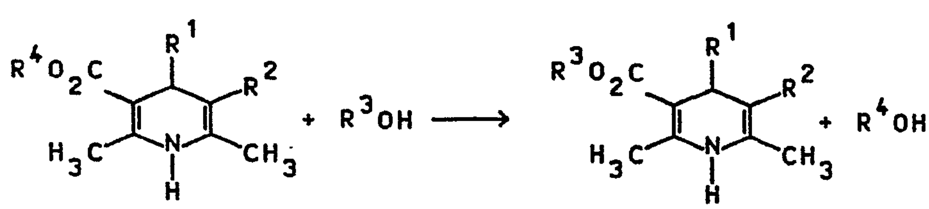

(Ia) (Ib)

Diese Umesterung wird in an sich bekannter Weise durchgeführt. Pro mol der Verbindung Ia wird mindestens 1 mol Alkohol der Formel $R^3OH$ eingesetzt. In der Regel werden pro mol Verbindung der Formel Ia 1,2 bis 50 mol, vorzugsweise 1,5 bis 30 mol $R^3OH$ eingesetzt und die Umesterung bei Normaltemperatur oder zweckmäßigerweise bei erhöhter Temperatur und zweckmäßigerweise in Anwesenheit eines Umesterungskatalysators durchgeführt. Sofern nicht überschüssiger Alkohol $R^3OH$ als Lösungsmittel dient, ist die Verwendung eines geeigneten inerten Lösungsmittels zweckmäßig. Geeignete inerte Lösungsmittel sind z.B.: Ether, insbesondere solche mit 2 bis 8 C-Atomen im Molekül, wie z.B. Diethylether, Methylethylether, Di-n-propylether, Di-iso-propylether, Methyl-n-butylether, Ethylpropylether, Dibutylether, Tetrahydrofuran, 1,4-Dioxan, 1,2-Dimethoxyethan, Bis-ß-methoxyethyl-ether; aliphatische Kohlenwasserstoffe, wie z.B. niedrig- und hochsiedende Petrolether; aromatische Kohlenwasserstoffe, wie z.B. Benzol, Toluol, o-, m- und p-Xylol, Pyridin; halogenierte aliphatische oder aromatische Kohlenwasserstoffe, wie z.B. Methylenchlorid, Tetrachlorkohlenstoff, Ethylenchlorid, Chlorbenzol, Dichlorbenzol. Auch Gemische verschiedener Lösungsmittel können verwendet werden.

Der zum Einsatz kommende Alkohol $R^3OH$ ergibt sich aufgrund der bereits genannten Bedeutungen für $R^3$. Somit kommt als Alkohol $R^3OH$ zum Einsatz: ein Alkanol mit 1 bis 6 C-Atomen, ein Alkoxyalkanol mit 3 bis 8 C-Atomen, ein Dialkylaminoalkanol mit insgesamt 4 bis 9 C-Atomen, ein N-Aralkyl-N-alkyl-aminoalkanol mit insgesamt 10 bis 14 C-Atomen, Cyclopentanol oder Cyclohexanol. Die bevorzugten Alkohole $R^3OH$ leiten sich entsprechend von den bevorzugten Resten $R^3$ und die besonders bevorzugten Alkohole $R^3OH$ von den besonders bevorzugten Resten $R^3$ ab. Beispiele für bevorzugte Alkohole $R^3OH$ sind: Methanol, Ethanol, N-Propanol, i-Propanol, n-Butanol, i-Butanol, sec-Butanol, tert-Butanol, Neopentanol, 2-Methoxy-ethanol; 2-i-Propoxy-ethanol, 2-n-Butoxy-ethanol, 3-Methoxy-n-propanol, 2-Dimethylamino-ethanol. Besonders bevorzugt werden Methanol, n-Propanol, n-Butanol, i-Butanol, tert-Butanol und 2-i-Propoxy-ethanol. Ganz besonders bevorzugt werden i-Propanol und 2-Methoxy-ethanol.

Als erhöhte Temperatur für die Umesterung kommen zunächst Temperaturen bis zur Rückflußtemperatur und - besonders bei Verwendung von überschüssigem Alkohol $R^3OH$ als Lösungsmittel - insbesondere die Rückflußtemperatur in Betracht. In manchen Fällen, insbesondere auch bei der Verwendung von überschüssigem Alkohol $R^3OH$ als Lösungsmittel, ist es jedoch zweckmäßig, die Umesterung bei einer Temperatur durchzuführen, die, z.B. bis zu 50°C oder mehr, über der Rückflußtemperatur bei Normaldruck liegt, weil dadurch die Ausbeute und/oder die Reinheit des Umesterungsproduktes zum Teil beträchtlich erhöht werden. Die Umesterung bei einer Temperatur, die gegenüber der Rückflußtemperatur erhöht ist, wird in einem Druckgefäß unter dem sich bei der Umesterung einstellenden Druck durchgeführt.

Unter Berücksichtigung der vorstehend genannten Gesichtspunkte wird die Umesterung im allgemeinen bei Temperaturen von 50 bis 190°C, vorzugsweise von 60 bis 150°C, durchgeführt.

Als geeignete Umesterungskatalysatoren kommen die bekannten Umesterungskatalysatoren in Betracht, so z.B. Säuren oder insbesondere Basen. Als Umesterungskatalysatoren geeignete Säuren sind z.B. anorganische Säuren, wie z.B. Halogenwasserstoffe, wie z.B. Chlorwasserstoff und Bromwasserstoff, Schwefelsäure, organische Sulfonsäuren, wie z.B. Benzol- oder p-Toluol-sulfonsäure, starke organische Säuren, wie z.B. Trifluoressigsäure, usw. Geeignete basische Umesterungskatalysatoren sind z.B. Alkali- oder Erdalkalisalze von Monocarbonsäuren, insbesondere solchen

mit 1 bis 4 C-Atomen, ferner Alkalihydroxide, wie z.B. Lithium-, Natrium- oder Kaliumhydroxid, und Alkalicarbonate, wie z.B. Natrium- oder Kaliumcarbonat. Weiter sind besonders geeignet die Alkali-, Erdalkali-, Aluminium-, Titan- und Germaniumverbindungen der Alkohole $R^3OH$, so z.B. die Natrium-, Kalium-, Lithium-, Magnesium-, Calcium-, Aluminium- oder Titanverbindungen der Alkohole $R^3OH$.

Diese Alkoholate müssen nicht unbedingt in isolierter Form eingesetzt werden, sondern können, wie z.B. im Fall der Alkali- oder Erdalkalialkoholate, häufig aus dem Alkohol $R^3OH$ und dem entsprechenden Alkali- oder Erdalkalimetall gebildet werden. In manchen Fällen können als Umesterungskatalysatoren auch Zinnsalze, Mangansalze, Zinn(IV)oxid und Antimontrioxid verwendet werden. Auch die Verwendung von Mischungen aus zwei oder mehreren geeigneten Umesterungskatalysatoren ist möglich.

Die Menge des zugesetzten Umesterungskatalysators wird so gering wie möglich gehalten. In der Regel genügen 0,01 bis 1,1 mol, vorzugsweise 0,02 bis 1,0 mol Umesterungskatalysator. Als Umesterungskatalysator werden die genannten basischen Umesterungskatalysatoren, insbesondere die Alkalisalze, vor allem die Lithium-, Natrium- oder Kaliumsalze der Alkohole $R^3OH$ bevorzugt. Ein besonders bevorzugter Alkohol $R^3OH$ für die Umesterung ist Isopropanol, insbesondere in Anwesenheit von Lithium-, Kalium- oder Natriumisopropanolat als Umesterungskatalysators. Ein weiterer besonders bevorzugter Alkohol $R^3OH$ für die Umesterung ist 2-Methoxy-ethanol, insbesondere in Anwesenheit von Lithium-, Kalium- oder Natrium-2-methoxyethanolat als Umesterungskatalysator.

Die bei der Umesterung erhaltene optisch aktive Verbindung Ib wird gewünschtenfalls in an sich bekannter Weise in ein Säureadditionssalz überführt.

Für die Herstellung der für die Umesterung benötigten Verbindungen der Formel Ia (Bedeutung von R = $R^4$ in der Formel I) in reiner, optisch aktiver Form kommen mehrere Verfahren in Betracht. Zweckmäßigerweise werden die substituierten, optisch aktiven 1,4-Dihydropyridine der Formel Ia in Analogie zu der Herstellung anderer 1,4-Dihydropyridin-Verbindungen dadurch hergestellt, daß

a) 1 mol einer Ylidenverbindung der Formel III

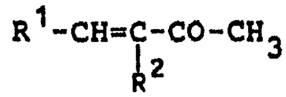 (III)

und 1 mol eines 3-Aminocrotonsäureesters der Formel IV

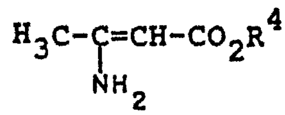 (IV)

oder daß

b) 1 mol eines Aldehyds der Formel V

$R^1-CHO$ (V),

1 mol eines 3-Aminocrotonsäureesters der Formel IV

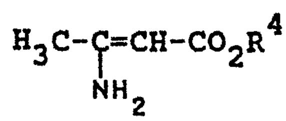 (IV)

und 1 mol eines Ketons der Formel VI

$R^2-CH_2-CO-CH_3$ (VI)

oder daß

c) 1 mol einer Ylidenverbindung der Formel III

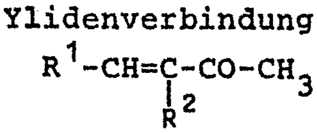 (III),

1 mol eines Acetessigesters der Formel VII

$H_3C-CO-CH_2-CO_2R^4$ (VII)

und 1 mol Ammoniak ($NH_3$)

oder daß

d) 1 mol einer Ylidenverbindung der Formel VIII

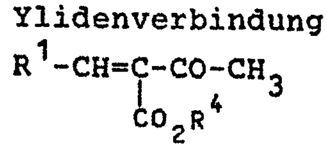 (VIII)

und 1 mol eines Enamins der Formel IX

$$R^2\text{-}CH{=}\underset{NH_2}{C}\text{-}CH_3 \qquad (IX)$$

oder daß

e) 1 mol eines Aldehyds der Formel V

$$R^1\text{-}CHO \qquad (V),$$

1 mol eines Acetessigesters der Formel VII

$$H_3C\text{-}CO\text{-}CH_2\text{-}CO_2R^4 \qquad (VII)$$

1 mol eines Ketons der Formel VI

$$R^2\text{-}CH_2\text{-}CO\text{-}CH_3 \qquad (VI)$$

und 1 mol Ammoniak ($NH_3$)

oder daß

f) 1 mol eines Aldehyds der Formel V

$$R^1\text{-}CHO \qquad (V),$$

1 mol eines Acetessigesters der Formel VII

$$H_3C\text{-}CO\text{-}CH_2\text{-}CO_2R^4 \qquad (VII)$$

und 1 mol eines Enamins der Formel IX

$$R^2\text{-}CH{=}\underset{NH^2}{C}\text{-}CO\text{-}CH_3 \qquad (IX)$$

miteinander umgesetzt werden, wobei in den Verbindungen der Formeln III bis IX $R^1$, $R^2$ und $R^4$ die eingangs genannten Bedeutungen besitzen, und daß aus den so erhaltenen Gemischen von Diastereomeren eine reine optisch aktive Verbindung isoliert und gegebenenfalls in ein Säureadditionssalz überführt wird.

Die Verbindungen der Formeln IV, VII und VIII sind aufgrund der Tatsache, daß der Rest $R^4$ optische Aktivität zeigt, selbst optisch aktiv. Bei der Herstellung der Verbindungen der Formel Ia werden die Verbindungen der Formeln IV, VII und VIII in Form der reinen optisch aktiven Verbindungen eingesetzt. Ausgehend von den Verbindungen der Formeln III bis IX bestehen aber noch weitere mögliche Syntheseverfahren für die Verbindungen der Formel Ia. Die Verfahrensvarianten stellen Varianten

oder Teilschritte der bekannten Hantzsch'schen Pyridinsynthese dar.

Die Umsetzung für die Herstellung der Verbindungen Ia wird bei allen Varianten a) bis f) bei Raumtemperatur (20°C) oder insbesondere erhöhter Temperatur, z.B. in einem Bereich von 20 bis 160°C, vorzugsweise 40 bis 120°C, durchgeführt. Normalerweise erfolgt die Umsetzung bei Normaldruck, kann aber auch bei einem vom Normaldruck abweichenden Druck durchgeführt werden.

Die Umsetzungen werden in Wasser oder einem inerten organischen Lösungsmittel durchgeführt. Geeignete Lösungsmittel sind z.B. Alkohole, insbesondere solche mit 1 bis 6 C-Atomen, wie z.B. Methanol, Ethanol, i- und n-Propanol, i-, sec.- und tert.-Butanol, n-, i-, sec.-, tert.-Pentanol, n-Hexanol, Cyclopentanol, Cyclohexanol; Ether, insbesondere solche mit 2 bis 8 C-Atomen im Molekül, wie z.B. Diethylether, Methyl-ethyl-ether, Di-n-propyl-ether, Di-iso-propyl-ether, Methyl-n-butyl-ether, Ethylpropylether, Di-butyl-ether, Tetrahydrofuran, 1,4-Dioxan, 1,2-Dimethoxyethan, Bis-ß-methoxyethyl-ether; Polyether, wie z.B. Polyethylenglykole mit einem Molekulargewicht bis ca. 600; Oligoethylen-glykol-dimethyl-ether, wie z.B. Pentaglyme; Glykole und teilweise veretherte Glykole, wie z.B. Ethylenglykol, Propylenglykol, Trimethylenglykol, Ethylenglykol-monomethyl-ether, Ethylenglykol-monoethylether, Diethylenglykol-monoethyl-ether; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-isopropylketon, Di-iso-butylketon, Cyclopentanon, Cyclohexanon, Benzophenon, Acetophenon; aliphatische Kohlenwasserstoffe, wie z.B. niedrig- und hochsiedende Petrolether; aromatische Kohlenwasserstoffe, wie z.B. Benzol, Toluol, o-, m- und p-Xylol, Pyridin; halogenierte aliphatische oder aromatische Kohlenwasserstoffe, wie z.B. Methylenchlorid, Chloroform, Tetrachlorkohlenstoff, Ethylenchlorid, Chlorbenzol, Dichlorbenzol; Nitrile, wie z.B. Acetonitril; Amide, wie z.B. Dimethylformamid, N-Methyl-pyrrolidon, Hexamethylphosphorsäuretriamid; Sulfoxide, wie z.B. Dimethylsulfoxid; Wasser. Auch Gemische verschiedener Lösungsmittel können verwendet werden. Alkohole oder Gemische von Alkoholen mit Wasser sind in der Regel bevorzugt.

Von den oben genannten Verfahrensvarianten für die Herstellung der Verbindungen der Formel Ia ist die Verfahrensvariante a) bevorzugt.

Die zur Herstellung der Verbindungen der Formel Ia benötigten Ausgangsprodukte der Formeln III bis IX sind bekannt oder können leicht nach den für die jeweilige Verbindungsklasse bekannten Verfahren hergestellt werden.

Die Verbindungen der Formel IX können, falls sie nicht bereits bekannt sind, z.B. hergestellt werden nach A.C. Cope, J. Amer. chem. Soc. 67, 1017 (1945). Als Beispiele für Enamino-Verbindungen der Formel IX seien genannt: 2-(2-Aminopropen-1-yl)-4-methyl-5-ethoxycarbonyl-thiazol, 2-(2-Aminopropen-1-yl)-thiazol, 2-(2-Aminopropen-1-yl)-4-phenyl-thiazol, 5-(2-Aminopropen-1-yl)-3-methyl-1,2,4-oxadiazol, 5-(2-Aminopropen-1-yl)-3-ethyl-1,2,4-oxadiazol, 5-(2-Aminopropen-1-yl)-3-tert.butyl-1,2,4-oxadiazol, 5-(2-Aminopropen-1-yl)-3-benzyl-1,2,4-oxadiazol, 2-(2-Aminopropen-1-yl)-1,3,4-oxadiazol, 2-(2-Aminopropen-1-yl)-5-aminocarbonylmethylthio-1,3,4-oxadiazol, 2-(2-Aminopropen-1-yl)-5-methyl-1,3,4-oxadiazol, 3-(2-Aminopropen-1-yl)-1,2,4-oxadiazol, 3-(2-Aminopropen-1-yl)-5-methyl-1,2,4-oxadiazol, 3-(2-Aminopropen-1-yl)-5-benzyl-1,2,4-oxadiazol, 5-(2-Aminopropen-1-yl)-1,2,4-thiadiazol, 5-(2-Aminopropen-1-yl)-3-methylthio-1,2,4-thiadiazol.

Die als Ausgangskomponenten dienenden Aldehyde der Formel V können, falls sie nicht bereits bekannt sind, z.B. hergestellt werden nach den von E. Mosettig, Org. Reactions VIII, 218 ff. (1954) beschriebenen Methoden. Beispiele für geeignete Aldehyde der Formel V sind: Benzaldehyd, 2-, 3- oder 4-Methyl-benzaldehyd, 2-, 3-oder 4-Ethyl-benzaldehyd, 2-, 3- oder 4-i-Propyl-benzaldehyd, 2-, 3- oder 4- tert.-Butyl-benzaldehyd, 2-, 3- oder 4-Methoxy-benzaldehyd, 2-, 3- oder 4-i-Propoxy-benzaldehyd, 2-, 3- oder 4-Brom-benzaldehyd, 2-, 3- oder 4-Chlor-benzaldehyd, 2-, 3- oder 4-Fluor-benzaldehyd, 2-, 3- oder 4-Cyano-benzaldehyd, 2-, 3- oder 4-Trifluormethyl-benzaldehyd, 2-, 3- oder 4-Nitro-benzaldehyd, 2,4- oder 2,6-Dimethyl-benzaldehyd, 2,4- oder 2,6-Dichlor-benzaldehyd, 2,4- oder 2,6-Dibrom-benzaldehyd, 2,4- oder 2,6-Dinitrobenzaldehyd, 2,4- oder 2,6-Diethylbenzaldehyd, 3-Chlor-4-trifluormethyl-benzaldehyd, 3-Methyl-4-trifluormethyl-benzaldehyd, 3-Methoxy-4-chlor-benzaldehyd, 2-Methyl-4-cyano-benzaldehyd, Pyridin-2-aldehyd, Pyridin-3-aldehyd, Pyridin-4-aldehyd, 4-Methyl- pyridin-2-aldehyd, 5-Methyl-pyridin-2-aldehyd, 6-Methyl- pyridin-2-aldehyd, Thiophen-2-aldehyd, Thiophen-3-aldehyd, 5-Nitro-thiophen-2-aldehyd, 5-Methyl-thiophen-2-aldehyd, 5-Chlor-thiophen-2-aldehyd, 5-Methoxy-thiophen-2-aldehyd.

Die als Ausgangsverbindungen der Formel VII benötigten reinen optisch aktiven Derivate der Acetessigsäure können, soweit sie nicht bereits bekannt sind, leicht durch Umsetzung eines (R)- oder (S)-Milchsäureesters der Formel (R)- oder (S)-$HO-CH(CH_3)CO_2R^5$ oder eines 2-O-($R^5$-carbonyl)-isosorbids der Formel IIa mit Diketen hergestellt werden. Derartige Umsetzungen sind in den nachfolgenden Beispielen beschrieben. Andere Verbindungen der Formel VII können analog hergestellt werden.

Die als Ausgangskomponenten benötigten Yliden-Verbindungen der Formeln III und VIII können, soweit sie nicht bereits bekannt sind, hergestellt werden nach Org.Reactions XV, 204 ff, (1967). Beispiele für geeignete Ausgangsverbindungen der Formel III sind: 1-(2,3-Dichlorphenyl)-2-(1,3,4-oxadiazol-2-yl)-1-buten-3-on, 1-(3-Nitrophenyl)-2-(1,3,4-oxadiazol-2-yl)-1-buten-3-on, 1-(2-Nitrophenyl)-2-(1,3,4-oxadiazol-2-yl)-1-buten-3-on, 1-(2-Chlorphenyl)-2-(1,3,4-oxadiazol-2-yl)-1-buten-3-on, 1-(3-Cyanophenyl)-2-(1,3,4-oxadiazol-2-yl)-1-buten-3-on, 1-Pyridin-3-yl)-2-(1,3,4-oxadiazol-2-yl)-1-buten-3-on, 1-(Thien-2-yl)-2-(1,3,4-oxadiazol-2-yl)-1-buten-3-on, 1-(3-Trifluormethylphenyl)-2-(1,3,4-oxadiazol-2-yl)-1-buten-3-on, 1-(2,3-Dichlorphenyl)-2-(2-methyl-1,3,4-oxadiazol-5-yl)-1-buten-3-on, 1-(2,3-Dichlorphenyl)-2-(2-benzyl-1,3,4-oxadiazol-5-yl)-1-buten-3-on, 1-(2-Methylphenyl)-2-(3-ethyl-1,2,4-oxadiazol-5-yl)-1-buten-3-on, 1-(3-Methoxyphenyl)-2-(3-tert.butyl-1,3,4-oxadiazol-5-yl)-1-buten-3-on, 1-(3-Chlorphenyl)-2-(1,2,4-thiadiazol-5-yl)-1-buten-3-on, 1-(2-Trifluormethyl)-2-(3-benzyl-1,2,4-oxadiazol-5-yl)-1-buten-3-on, 1-(2,5-Dichlorphenyl)-2-(3-methylthio-1,3,4-oxadiazol)-1-buten-3-on, 1-(Pyridin-2-yl)-2-(4-methyl-5-ethoxycarbonyl-thiazol-2-yl)-1-buten-3-on, 1-(3-Nitrophenyl)-2-(3-methyl-1,2,4-oxadiazol-5-yl)-1-buten-3-on, 1-(2,3-Dichlorphenyl)-2-(3-ethyl-1,2,4-oxadiazol-5-yl)-1-buten-3-on.

Die Yliden-Verbindungen der Formel VIII können in reiner optisch aktiver Form dadurch hergestellt werden, daß die vorstehend erwähnten optisch aktiven Verbindungen der Formel VII mit Aldehyden der Formel V nach bekannten Verfahren kondensiert werden.

Die 3-Aminocrotonsäureesterderivate der Formel IV können leicht in reiner optisch aktiver Form dadurch hergestellt werden, daß reine optisch aktive Acetessigesterderivate der Formel VII in an sich bekannter Weise mit Ammoniak in

die Aminocrotonsäurederivate überführt werden. Derartige Umsetzungen sind in den nachfolgenden Beispielen beschrieben. Andere Verbindungen der Formel IV können analog hergestellt werden.

Die Ketone der Formel VI können, soweit sie nicht bereits bekannt sind, nach dem in Monatshefte für Chemie 113, 781 ff. (1982) beschriebenen Verfahren hergestellt werden. Geeignete Ausgangsverbindungen der Formel VI sind z.B. 5-Acetonyl-1,2,4-oxadiazol, 3-Methyl-5-acetyl-1,2,4-oxadiazol, 3-Ethyl-5-acetyl-1,2,4-oxadiazol, 3-tert.Butyl-5-acetyl-1,2,4-oxadiazol, 3-Methylthio-5-acetyl-1,2,4-oxadiazol, 3-Benzyl-5-acetyl-1,2,4-oxadiazol, 2-Acetonyl-1,3,4-oxadiazol, 5-Methyl-2-acetonyl-1,3,4-oxadiazol, 5-i-Propyl-2-acetonyl-1,3,4-oxadiazol, 3-Acetonyl-1,2,4-oxadiazol, 5-Ethyl-3-acetonyl-1,2,4-oxadiazol, 5-Ethylthio-3-acetonyl-1,2,4-oxadiazol, 5-Phenethyl-3-acetonyl-1,2,4-oxadiazol, 5-Acetonyl-1,2,4-thiadiazol, 3-Ethyl-5-acetonyl-1,2,4-thiadiazol, 3-Benzyl-5-acetonyl-1,2,4-thiadiazol.

Die nach den Verfahren a) bis f) herstellbaren Verbindungen der Formel Ia besitzen im Rest $R^4$ und in der 4-Stellung des Dihydropyridinkerns asymmetrische Kohlenstoffatome und entstehen daher nach den Verfahren a) bis f) in Form von Gemischen von Diastereomeren. Überraschenderweise hat sich gezeigt, daß bei der praktischen Durchführung der Verfahren a) bis f) Rohgemische von Diastereomeren erhalten werden, in denen jeweils ein Diastereomeres im Überschuß vorhanden ist. Aufgrund der unterschiedlichen Verteilung der Diastereomeren in den erhaltenen Rohgemischen und der unterschiedlichen physikalischen Eigenschaften der Diastereomeren kann aus den bei den Umsetzungen a) bis f) erhaltenen Rohgemischen ein Distereomeres in optisch reiner Form isoliert werden. Für diese Isolierung steht eine Reihe von Trenn- oder Reinigungsverfahren, wie z.B. Chromatographie, Extraktion oder Umkristallisation zur Verfügung. Je nach dem angewandten

Trennverfahren und den physikalischen Eigenschaften der Diastereomeren läßt sich entweder das im Unterschuß oder das im Überschuß vorhandene Diastereomere leichter isolieren. Es ist auch möglich, beide Diastereomeren in reiner Form zu isolieren. In der Regel erfolgt die Isolierung eines im Rohgemisch vorhandenen Diastereomeren der Formel Ia in optisch aktiver reiner Form durch Umkristallisation, wobei im allgemeinen das im Rohgemisch als Hauptprodukt im Überschuß vorhandene Diastereomere isoliert wird. Für die Isolierung der Verbindung in reiner optisch aktiver Form ist in der Regel hierzu nur ein einmaliges Umkristallisieren erforderlich. Für diese Umkristallisation kommen die üblichen Lösungsmittel in Betracht, insbesondere Alkohole mit 1 bis 4 C-Atomen, wie z.B. Methanol, Ethanol, Propanol, Isopropanol, Butanol, i-Butanol; Ketone, wie z.B. Aceton, Methylethylketon; Ether, wie z.B. Diethylether; Glykolether, wie z.B. Ethylenglykolmonomethylether, Ethylenglykoldimethylether, oder Gemische verschiedener Lösungsmittel, insbesondere auch mit Wasser.

Für den Fall, daß bei der Umesterung mit dem Alkohol $R^3OH$ die Verbindung der Formel Ia in reiner optisch aktiver Form eingesetzt wird, erhält man bei der Umesterung die Verbindung der Formel Ib ebenfalls in reiner optisch aktiver Form und zwar ausgehend von Verbindungen Ia mit $R^4$ = (S)-$CH(CH_3)COOR^5$ in der Regel rechtsdrehende Isomeren der Formel Ib und ausgehend von Verbindungen Ia mit $R^4$ = (R)-$CH(CH_3)COOR^5$ in der Regel linksdrehende Isomeren der Formel Ib, und ausgehend von Verbindungen der Formel Ia mit $R^4$ = 2-O-($R^5$-carbonyl)-isosorbid-5-yl in der Regel die linksdrehenden Isomeren der Formel Ib.

Die optisch aktiven 1,4-Dihydropyridin-Derivate der Formel I bilden, sofern sie basische Substituenten besitzen, mit anorganischen oder organischen Säuren Säureadditionssalze. Zur Bildung derartiger Säureadditionssalze sind

anorganische und organische Säuren geeignet. Geeignete Säuren sind beispielsweise: Chlorwasserstoff, Bromwasserstoff, Naphthalindisulfonsäuren, insbesondere Naphthalin-1,5-disulfonsäure, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipin-Säure. Pharmakologisch annehmbare Säureadditionssalze werden bevorzugt. Die Säureadditionssalze werden wie üblich durch Vereinigung der Komponenten, zweckmäßigerweise in einem geeigneten Lösungs- oder Verdünnungsmittel, hergestellt. Bei der Synthese der Verbindungen der Formel I können zunächst die Säureadditionssalze im Zuge der Aufarbeitung anfallen. Aus den Säureadditionsalzen können die freien Verbindungen der allgemeinen Formel I gewünschtenfalls in bekannter Weise, z.B. durch Auflösen oder Suspendieren in Wasser und Alkalischstellen, z.B. mit Natronlauge, und anschließendes Isolieren, gewonnen werden.

Die in der europäischen Patentanmeldung 83 112 562.0 beschriebenen, optisch inaktiven Verbindungen der Formel Ib besitzen interessante kardiovaskuläre Wirkungen. Als hochwirksame Calciumantagonisten hemmen sie die durch Calcium in der Muskelzelle hervorgerufene Kontraktion und besitzen eine blutdrucksenkende und antianginöse Wirkung und können so z.B. zur Blutdrucksenkung und Herzentlastung beitragen.

Es wurde nun gefunden, daß die pharmakologische Wirkung der Verbindungen der Formel I in hohem Maße an die Konfiguration der chemischen Verbindungen gekoppelt ist, und zwar sind in der Regel die linksdrehenden optischen Isomeren etwa um den Faktor 100 wirksamere Calciumantagonisten als die entsprechenden rechtsdrehenden Enantiomeren.

Die linksdrehenden optischen Isomeren sind daher in der Regel bevorzugt.

Die optisch aktiven Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon neben üblichen pharmazeutisch einwandfreien Träger- und Zusatzstoffen enthalten. Die Zubereitungen enthalten normalerweise etwa 0,5 bis 90 Gewichtsprozent der therapeutisch wirksamen Verbindung.

Die Heilmittel können oral, z.B. in Form von Pillen, Tabletten, Lacktabletten, Dragees, Granulaten, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole,

Glyzerin, Polyole, pflanzliche Öle etc.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I und/oder ihrer pharmakologisch annehmbaren Säureadditionssalze und noch andere therapeutisch wirksame Stoffe enthalten.

Derartige andere therapeutisch wirksame Substanzen sind beispielsweise: ß-Rezeptorenblocker, wie z.B. Propranolol, Pindolol, Metoprolol; antianginöse Mittel, wie z.B. Carbochromen oder Molsidomin; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin; Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Verbindungen der Formel I, ihre pharmakologisch annehmbaren Säureadditionssalze und pharmazeutische Präparate, welche die Verbindungen der Formel I oder ihre pharmakologisch annehmbaren Säureadditionssalze als Wirkstoff enthalten, können am Menschen bei der Bekämpfung bzw. Vorbeugung von Erkrankungen verwendet werden, die durch ein Einströmen von Calcium in die Muskelzellen hervorgerufen werden und die durch die Verabreichung von

Calciumantagonisten bekämpft werden können. So können sie beispielsweise als antihypertensive Heilmittel bei den verschiedenen Formen des Bluthochdrucks, bei der Bekämpfung bzw. Vorbeugung von Angina pectoris usw., sowie bei der Behandlung von cerebralen und peripheren Durchblutungsstörungen eingesetzt werden. Die Dosierung kann innerhalb weiter Grenzen variieren und ist in jedem einzelnen Fall den individuellen Gegebenheiten anzupassen. Im allgemeinen ist bei oraler Verabreichung eine Tagesdosis von etwa 0,01 bis 10mg/kg, vorzugsweise 0,05 bis 5mg/kg, Körpergewicht zur Erzielung wirksamer Ergebnisse angemessen. Bei intravenöser Applikation beträgt die Tagesdosis im allgemeinen etwa 0,001 bis 10 mg/kg, vorzugsweise 0,01 bis 5 mg/kg, Körpergewicht. Die Tagesdosis wird normalerweise, insbesondere bei der Applikation größerer Mengen, in mehrere, z.B. 2, 3 oder 4, Teilverabreichungen aufgeteilt. Gegebenenfalls kann es je nach individuellem Verhalten erforderlich werden, von der angegebenen Tagesdosis nach oben oder unten abzuweichen.

Bei dem in den nachfolgenden Beispielen angegebenen Wert $\alpha_D^{20}$ handelt es sich um die spezifische Drehung der Substanz für polarisiertes Licht der Natrium-D-Linie (589nm) bei 20°C. Nach dem angegebenen Wert für die spezifische Drehung sind in den Beispielen in Klammern das bei der Messung benutzte Lösungsmittel und die Konzentration c in g/100ml angegeben.

Beispiel 1

(-)1,4-Dihydro-2,6-dimethyl-3-(1,3,4-oxadiazol-2-yl)-4-(2,3-dichlorphenyl)-pyridin-5-carbonsäure-isopropylester

a) Isosorbid-2-acetat-5-acetylacetat

188 g Isosorbid-2-acetat werden in 400 ml Methylenchlorid gelöst, auf 35°C vorgewärmt und mit 10 ml Triethylamin versetzt. In dieser Mischung werden 84 g Diketen so zugetropft, daß die Innentemperatur zwischen 40 und 45°C beträgt. Die Mischung wird dann über Nacht stehengelassen und am Rotationsverdampfer bei 40°C eingeengt.
Ausbeute: 289 g Öl.

b) Isosorbid-2-acetat-5-aminocrotonat

289 g rohes Isosorbid-2-acetat-5-acetylacetat werden im Eisbad auf 5 bis 10°C vorgekühlt. Unter Umrühren wird nun eine auf 10°C vorgekühlte Lösung von 20g Ammoniak in 250 ml Ethanol so zugefügt, daß die Innentemperatur unterhalb von 25°C bleibt. Dann wird 2 h im Eisbad nachgerührt, wobei die Verbindung auskristallisiert. Sie wird abgesaugt und aus 500 ml Isopropanol umkristallisiert.
Ausbeute: 182 g, Fp = 120°C.

c) 2-(1-(2,3-Dichlorbenzyliden)-acetonyl)-1,3,4-oxadiazol

70 g 2-Acetonyl-1,3,4-oxadiazol und 92 g 2,3-Dichlorbenzaldehyd werden in 300 ml Toluol gelöst und mit 5 ml Piperidin versetzt. Die Mischung wird am Wasserabscheider erhitzt, bis sich kein Wasser mehr abscheidet. Das Toluol wird im Vakuum abgezogen und der Rückstand aus 250 ml Isopropanol umkristallisiert.
Ausbeute: 98 g, Fp = 98 bis 100°C.

d) (-)1,4-Dihydro-2,6-dimethyl-3-(1,3,4-oxadiazol-2-yl)-4-(2,3-dichlorphenyl)-pyridin-5-carbonsäure-(2-O-acetyl-iso-sorbid-5-yl)-ester

134 g 2-(1-(2,3-Dichlorbenzyliden)-acetonyl)-1,3,4-oxadiazol und 128 g Isosorbid-2-acetat-5-aminocrotonat werden in 300 ml Dimethylformamid 12 h auf 80°C erwärmt. Die Mischung wird im Vakuum bei 80°C eingeengt und der Rückstand aus 500 ml Isopropanol umkristallisiert.
Ausbeute: 138 g , Fp= 255 bis 258°. $\alpha_D^{20} = -45°$ ($CH_2Cl_2$, c = 2,0). Aus der Mutterlage läßt sich noch weiteres Produkt isolieren.

e) (-)1,4-Dihydro-2,6-dimethyl-3-(1,3,4-oxadiazol-2-yl)-4-(2,3-dichlorphenyl)-pyridin-5-carbonsäure-isopro-

pylester

44,5 g (-)1,4-Dihydro-2,6-dimethyl-3-(1,3,4-oxadiazol-2-yl)-4-(2,3-dichlorphenyl)-pyridin-5-carbonsäure-(2-acetyl-iso-sorbid-5-yl)-ester werden zur Lösung von 2 g Natrium in 150 ml absolutem Isopropanol gegeben und die Mischung 15 h am Rückfluß gekocht. Die Mischung wird im Wasserstrahlvakuum eingeengt, der Rückstand auf 500 ml Eiswasser gegossen und die Verbindung mit 500 ml Essigsäureethylester extrahiert. Die Essigesterphase wird einmal mit einem Gemisch aus 200 ml Wasser und 10ml conz. Salzsäure und dann mit 200 ml Wasser ausgeschüttelt, über $Na_2SO_4$ getrocknet und eingeengt. Der Rückstand wird zweimal aus Isopropanol umkristallisiert.
Ausbeute: 12 g, Fp = 199 bis 202°C. $\alpha_D^{20}$ = -95° ($CH_2Cl_2$; c = 2,0).

In analoger Weise lassen sich herstellen:

Beispiel 2:
(-)1,4-Dihydro-2,6-dimethyl-3-(1,3,4-oxadiazol-2-yl)-4-(2,3-dichlorphenyl)-pyridin-5-carbonsäure-methylester
Fp = 225 bis 229°C, $\alpha_D^{20}$ = -101° ($CH_2Cl_2$; c = 1,7).

Beispiel 3:
(-)1,4-Dihydro-2,6-dimethyl-3-(1,3,4-oxadiazol-2-yl)-4-(2,3-dichlorphenyl)-pyridin-5-carbonsäure-ethylester
Fp = 187-189°C. $\alpha_D^{20}$ = -90° ($CH_2Cl_2$; c = 2,0)

Beispiel 4:
(-)1,4-Dihydro-2,6-dimethyl-3-(1,3,4-oxadiazol-2-yl)-4-(2,3-dichlorphenyl)-pyridin-5-carbonsäure-methoxyethylester
Fp = 192-195°C. $\alpha_D^{20}$ = -60° ($CH_2Cl_2$; c = 1,0)

Beispiel 5:
(+)1,4-Dihydro-2,6-dimethyl-3-(1,3,4-oxadiazol-2-yl)-4-(2,3-dichlorphenyl)-pyridin-5-carbonsäure-isopropylester

a) (-)O-Acetylacetyl-(S)-milchsäure-ethylester

118 g (S)Milchsäure-ethylester und 5 ml Triethylamin werden in 300 ml Methylenchlorid gelöst. 84 g Diketen werden so langsam zugetropft, daß die Mischung schwach siedet. Nach beendeter Zugabe läßt man 15 h bei Raumtemperatur stehen, engt im Wasserstrahlvakuum ein und destilliert:
Kp 140 bis 145°C / 26,7 mbar; Ausbeute: 162,9 g.
$\alpha_D^{20}$ = -33° ($CH_2Cl_2$; c = 2,0).

b) (-)Aminocrotonsäure-((S)-1-ethoxycarbonyl-ethyl)-ester

80 g (-)O-Acetylacetyl-(S)-milchsäure-ethylester werden in einer Lösung von 20 g Ammoniak in 250 ml Ethanol gelöst und 20 h bei Raumtemperatur stehen gelassen. Nach dem Einengen im Wasserstrahlvakuum bei 40°C wird destilliert:
Kp 130 bis 135°C /2mbar. Ausbeute 53 g.
$\alpha_D^{20}$ = -77,5° ($CH_2Cl_2$; c = 2,0).

c) (+)1,4-Dihydro-2,6-dimethyl-3-(1,3,4-oxadiazol-2-yl)-4-(2,3-dichlorphenyl)-pyridin-5-carbonsäure-((S)-1-ethoxycarbonyl-ethyl)ester

30 g 2-(1(2,3-Dichlorbenzyliden)-acetonyl)-1,3,4-oxadiazol und 21,3 g (-)Aminocrotonsäure-((S)-1-ethoxycarbonyl-ethyl)-ester werden in 100 ml Dimethylsulfoxyd 15 h auf 80°C erwärmt. Die Reaktionsmischung wird auf 300 ml Eiswasser gegossen, wobei ein Niederschlag ausfällt, der nach 1 h Nachrühren abgesaugt und aus 500 ml Ethanol umkristallisiert wird: Ausbeute: 28 g, Fp = 218 bis 220°C, $\alpha_D^{20}$ = +98° ($CH_2Cl_2$; c = 2,0).

d) (+)1,4-Dihydro-2,6-dimethyl-3-(1,3,4-oxadiazol-2-yl-)4-(2,3-dichlorphenyl)-pyridin-5-carbonsäure-isopropyl-ester

Zur Lösung von 2,2 g Natrium in 400 ml Isopropanol werden 44 g (+)1,4-Dihydro-2,6-dimethyl-3-(1,3,4-oxadiazol-2-yl)-4-(2,3-dichlorphenyl)-pyridin-5-carbonsäure-((S)-1-ethoxycarbonyl-ethyl)-ester gegeben und die Mischung 20 h am Rückfluß erhitzt. Die Mischung wird auf ein Volumen von etwa 150 ml im Wasserstrahlvakuum eingeengt und auf 600 ml Eiswasser gegossen. Das Produkt wird mit dreimal je 200 ml Diethylether extrahiert, die vereinigten Etherphasen werden noch zweimal mit je 300 ml Wasser gewaschen, über Natriumsulfat getrocknet und im Wasserstrahlvakuum eingeengt. Der Rückstand wird mit wenig Diethylether verrührt und nach beendeter Kristallisation abgesaugt. Der Feststoff wird aus Ethanol/Wasser umkristallisiert:
Ausbeute: 10,3 g, Fp = 198 bis 201°C. $\alpha_D^{20}$ = +90° ($CH_2Cl_2$; c = 3,8).

In analoger Weise lassen sich herstellen:

Beispiel 6:
(+)1,4-Dihydro-2,6-dimethyl-3-(1,3,4-oxadiazol-2-yl)-4-(2,3-dichlorphenyl)-pyridin-5-carbonsäure-methylester
Fp = 225-228°C. $\alpha_D^{20}$ = +105,7° ($CH_2Cl_2$; c = 1,0).

Beispiel 7:
(+)1,4-Dihydro-2,6-dimethyl-3-(1,3,4-oxadiazol-2-yl)-4-(2,3-dichlorphenyl)-pyridin-5-carbonsäure-(2-methoxyethyl)-ester
Fp = 190 - 192°C. $\alpha_D^{20}$ = +73° ($CH_2Cl_2$; c = 0,4).

Beispiel 8:
(-)1,4-Dihydro-2,6-dimethyl-3-(1,3,4-oxadiazol-2-yl)-4-(2,3-dichlorphenyl)-pyridin-5-carbonsäure-isopropylester

a) (+)O-Acetylacetyl-(R)-milchsäure-ethylester
118 g (R)Milchsäure-ethylester und 5 ml Triethylamin werden in 300 ml Methylenchlorid gelöst. 84 g Diketen werden so langsam zugetropft, daß die Mischung schwach siedet.

Nach beendeter Zugabe läßt man 15 h bei Raumtemperatur stehen, engt im Wasserstrahlvakuum ein und destilliert: Kp 140 bis 145°C / 26,7 mbar; Ausbeute: 165 g. $\alpha_D^{20}$ = +35° ($CH_2Cl_2$; c = 2,0).

b) <u>(+)Aminocrotonsäure-((R)-1-ethoxycarbonyl-ethyl)-ester</u>
80 g (+)O-Acetylacetyl-(R)-milchsäure-ethylester werden in einer Lösung von 20 g Ammoniak in 250 ml Ethanol gelöst und 20 h bei Raumtemperatur stehen gelassen. Nach dem Abkühlen fällt ein kristalliner Niederschlag aus. Fp = 61 - 63°C, Ausbeute 65 g. $\alpha_D^{20}$ = +75° ($CH_2Cl_2$; c = 2,0).

c) (-)1,4-Dihydro-2,6-dimethyl-3-(1,3,4-oxadiazol-2-yl)-4-(2,3-dichlorphenyl)-pyridin-5-carbonsäure-((R)-1-ethoxycarbonyl-ethyl)ester
30 g 2-(1(2,3-Dichlorbenzyliden)-acetonyl)-1,3,4-oxadiazol und 21,3 g (+)Aminocrotonsäure-((R)-1-ethoxycarbonyl-ethyl)-ester werden in 100 ml Dimethylsulfoxyd 15 h auf 80°C erwärmt. Die Reaktionsmischung wird auf 300 ml Eiswasser gegossen, wobei ein Niederschlag ausfällt, der nach 1 h Nachrühren abgesaugt und aus 500 ml Ethanol umkristallisiert wird: Ausbeute: 28 g, Fp = 218 bis 220°C, $\alpha_D^{20}$ = -100° ($CH_2Cl_2$; c = 2,0).

d) (-)1,4-Dihydro-2,6-dimethyl-3-(1,3,4-oxadiazol-2-yl)-4-(2,3-dichlorphenyl)-pyridin-5-carbonsäure-isopropyl-ester
Zur Lösung von 150 mg Lithium in 100 ml Isopropanol werden 10 g (-)1,4-Dihydro-2,6-dimethyl-3-(1,3,4-oxadiazol-2-yl)-4-(2,3-dichlorphenyl)-pyridin-5-carbonsäure-((R)-1-ethoxycarbonyl-ethyl)-ester gegeben und die Mischung 2 h im Autoklaven auf 110°C erhitzt. Die Mischung wird auf 1/3 des ursprünglichen Volumens im Wasserstrahlvakuum eingeengt und auf 150 ml Eiswasser gegossen. Das feste Produkt wird abgesaugt und aus Ethanol/Wasser (Volumenverhältnis 6:4) umkristallisiert:
Ausbeute: 5,5 g, Fp = 202 bis 204°C. $\alpha_D^{20}$ = -96°

($CH_2Cl_2$; c = 2,0).

In analoger Weise lassen sich herstellen:

Beispiel 9:
(-)1,4-Dihydro-2,6-dimethyl-3-(1,3,4-oxadiazol-2-yl)-4-(2,3-dichlorphenyl)-pyridin-5-carbonsäure-methylester

Beispiel 10:
(-)1,4-Dihydro-2,6-dimethyl-3-(1,3,4-oxadiazol-2-yl)-4-(2,3-dichlorphenyl)-pyridin-5-carbonsäure-ethylester

Beispiel 11:
(-)1,4-Dihydro-2,6-dimethyl-3-(1,3,4-oxadiazol-2-yl)-4-(2,3-dichlorphenyl)-pyridin-5-carbonsäure-propylester

Beispiel 12:
(-)1,4-Dihydro-2,6-dimethyl-3-(1,3,4-oxadiazol-2-yl)-4-(2,3-dichlorphenyl)-pyridin-5-carbonsäure-butylester

Beispiel 13:
(-)1,4-Dihydro-2,6-dimethyl-3-(1,3,4-oxadiazol-2-yl)-4-(2,3-dichlorphenyl)-pyridin-5-carbonsäure-isobutylester

Beispiel 14:
(-)1,4-Dihydro-2,6-dimethyl-3-(1,3,4-oxadiazol-2-yl)-4-(2,3-dichlorphenyl)-pyridin-5-carbonsäure-(2-methoxy-ethyl)-ester

Beispiel 15:
(-)1,4-Dihydro-2,6-dimethyl-3-(1,3,4-oxadiazol-2-yl)-4-(2,3-dichlorphenyl)-pyridin-5-carbonsäure-(3-methoxy-propyl)-ester

Beispiel 16:
(-)1,4-Dihydro-2,6-dimethyl-3-(1,3,4-oxadiazol-2-yl)-4-(2,3-dichlorphenyl)-pyridin-5-carbonsäure-(2-isopropoxy-

ethyl)-ester

Fp = 151 - 154°C. $\alpha_D^{20}$ = -50° ($CH_2Cl_2$; c = 2,0).

Beispiel 17:

(-)1,4-Dihydro-2,6-dimethyl-3-(1,3,4-oxadiazol-2-yl)-4-(2,3-dichlorphenyl)-pyridin-5-carbonsäure-cyclopentyl-ester

Beispiel 18:

(-)1,4-Dihydro-2,6-dimethyl-3-(1,3,4-oxadiazol-2-yl)-4-(2,3-dichlorphenyl)-pyridin-5-carbonsäure-(2-diethylamino-ethyl)-ester

Fp = 142-144°C. $\alpha_D^{20}$ = -30° ($CH_2Cl_2$; c = 2,0).

Beispiel 19:

(-)1,4-Dihydro-2,6-dimethyl-3-(1,3,4-oxadiazol-2-yl)-4-(2,3-dichlorphenyl)-pyridin-5-carbonsäure-(2-(N-benzyl-N-methyl-amino)-ethyl)-ester

Beispiel 20:

(-)1,4-Dihydro-2,6-dimethyl-3-(1,3,4-oxadiazol-2-yl)-4-(2,3-dichlorphenyl)-pyridin-5-carbonsäure-isoamylester

Beispiel 21:

(-)1,4-Dihydro-2,6-dimethyl-3-(1,3,4-oxadiazol-2-yl)-4-(2,3-dichlorphenyl)-pyridin-5-carbonsäure-cyclohexylester

Beispiel 22

(-)1,4-Dihydro-2,6-dimethyl-3-(1,3,4-oxadiazol-2-yl)-4-2,3-dichlorphenyl)-pyridin-5-carbonsäure-neopentylester

Beispiel 23

(-)1,4-Dihydro-2,6-dimethyl-3-(1,3,4-oxadiazol-2-yl)-4-(2,3-dichlorphenyl)-pyridin-5-carbonsäure-isopropylester

Die ethanolische Mutterlauge des Beispiels 5c wird im Vakuum eingeengt. Der Rückstand wird in 150 ml Diethylether aufgenommen und 3 Tage bei Raumtemperatur und anschließend 4 Stunden bei 0°C gerührt. Der ausgefallene Niederschlag wird abfiltriert und verworfen, die etherische Lösung wird eingeengt, wobei 20 g eines gelben Öls verbleiben, das (+)1,4-Dihydro-2,6-dimethyl-3-(1,3,4-oxadiazol-2-yl)-4-((2,3-dichlorphenyl)-pyridin-5-carbonsäure-((S)-1-ethoxycarbonyl-ethyl)ester ist,
$\alpha_D^{20}$ = +27,7° ($CH_2Cl_2$; c = 2,0).

Dieses Produkt wird wie im Beispiel 5d angegeben umgeestert und gibt 7,5 g Feststoff.
Fp = 199 - 201°C. $\alpha_D^{20}$ = -91° ($CH_2Cl_2$; c = 2,0).

In den nachfolgenden Beispielen werden pharmazeutische Präparate beschrieben:

<u>Beispiel 24:</u>
Gelatineweichkapseln, enthaltend 5 mg Wirkstoff pro Kapsel:

| | pro Kapsel |
|---|---|
| Wirkstoff der Formel I | 5 mg |
| Aus Kokosfett fraktioniertes Triglyceridgemisch | 150 mg |
| Kapselinhalt | 155 mg |

<u>Beispiel 25:</u>
Injektionslösung, enthaltend 1 mg Wirkstoff pro mol:

| | | pro ml |
|---|---|---|
| Wirkstoff der Formel I | | 1,0 mg |
| Polyethylenglycol | | 0,3 ml |
| Natriumchlorid | | 2,7 mg |
| Wasser zu Injektionszwecken | ad | 1,0 ml |

<u>Beispiel 26:</u>
Emulsion, enthaltend 10 mg Wirkstoff pro 5 ml:

| | pro 100 ml |
|---|---|
| Wirkstoff der Formel I | 0,2 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

Beispiel 27:

Rektale Arzneiform, enthaltend 8 mg Wirkstoff pro Suppositorium

| | pro Suppositorium |
|---|---|
| Wirkstoff der Formel I | 8 mg |
| Suppositoriengrundmasse | ad 2 g |

Beispiel 28:

Tabletten, enthaltend 5 mg Wirkstoff pro Tablette

| | pro Tablette |
|---|---|
| Wasserstoff der Formel I | 5 mg |
| Maisstärke (weiß) | 150 mg |
| Milchzucker | 60 mg |
| Mikrokristalline Cellulose | 50 mg |
| Polyvinylpyrrolidon | 20 mg |
| Magnesiumstearat | 2 mg |
| Natriumcarboxymethylstärke | 25 mg |
| | 312 mg |

Beispiel 29:

Dragees, enthaltend einen erfindungsgemäßen Wirkstoff und einen anderen therapeutisch wirksamen Stoff

| | pro Dragee |
|---|---|
| Wirkstoff der Formel I | 6 mg |
| Propanolol | 40 mg |
| Milchzucker | 90 mg |
| Maisstärke | 90 mg |
| sec.-Calciumphosphat | 34 mg |
| lösliche Stärke | 3 mg |
| Magnesiumstearat | 3 mg |
| kolloidale Kieselsäure | 4 mg |
| | 270 mg |

Beispiel 30

Dragees, enthaltend einen erfindungsgemäßen Wirkstoff und einen anderen therapeutisch wirksamen Stoff

| | pro Dragee |
|---|---|
| Wirkstoff der Formel I | 6 mg |
| Molsidomin | 5 mg |
| Milchzucker | 90 mg |
| Maistärke | 90 mg |
| sec.-Calciumphosphat | 34 mg |
| lösliche Stärke | 3 mg |
| Magnesiumstearat | 3 mg |
| kolloidale Kieselsäure | 4 mg |
| | 235 mg |

Beispiel 31:

Kapseln, enthaltend einen erfindungsgemäßen Wirkstoff und einen anderen therapeutisch wirksamen Stoff

| | pro Kapsel |
|---|---|
| Wirkstoff der Formel I | 10 mg |
| Prazosin | 5 mg |
| Maisstärke | 185 mg |
| | 200 mg |

Die calciumantagonistische Wirkung der Verbindungen der Formel I wurde nach einer modifizierten Methode von Godfraind and Kaba (Arch. Int. Pharmacodyn. Ther. 196, (Suppl) 35 bis 49, 1972) und von Schümann et al (Naunyn-

Schmiedeberg's Arch. Pharmacol. 289, 409 bis 418, 1975) ermittelt. Dabei werden Spiralstreifen der Arteria pulmonalis des Meerschweinchens nach Äquilibrierung in calciumfreier Tyrodelösung mit 40mmol Kalium depolarisiert. Ein Zusatz von 0,5 mmol $CaCl_2$ löst dann die Kontraktion aus. Die relaxierende Wirkung der Prüfsubstanz wird durch kumulative Zugabe in 1/2 log 10 abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50% hemmt (= $IC_{50}$, mol/l). In der folgenden Tabelle sind die so erhaltenen $IC_{50}$-Werte angegeben. Wie der Vergleich mit dem $IC_{50}$-Wert $3 \cdot 10^{-9}$ für die bekannte Verbindung Nifedipin (=1,4-Dihydro-2,6-dimethyl-4-(2-nitrophenyl)-pyridin-3,5-di-(carbonsäuremethylester)), vgl. DE-B-16 70 827, ergibt, liegen die Werte für die Verbindungen der Formel I z.T. erheblich günstiger.

Tabelle

| Verbindung der Formel I gemäß Beispiel | $IC_{50}$(mol/l) |
|---|---|
| 1e | $4.10^{-11}$ |
| 2 | $2.10^{-10}$ |
| 3 | $7.10^{-11}$ |
| 4 | $3.10^{-10}$ |
| 8c | $4.10^{-11}$ |
| 16 | $1.10^{-10}$ |

P A T E N T A N S P R Ü C H E

1. Verfahren zur Herstellung optisch aktiver, substituierter 1,4-Dihydropyridine der Formel Ia

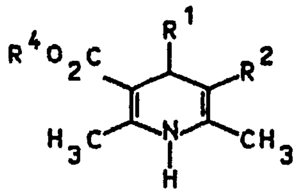

(Ia)

und ihrer Säureadditionssalze, wobei in der Formel Ia

$R^1$ Pyridyl oder Thienyl, wobei der Pyridyl- oder Thienyl-Rest gegebenenfalls 1 oder 2 gleiche oder verschiedene Substituenten aus der Gruppe Alkyl mit 1 bis 4 C-Atomen, Alkoxy mit 1 bis 4 C-Atomen, Halogen, Trifluormethyl, Nitro, Cyano besitzt; Phenyl, das gegebenenfalls 1 oder 2 gleiche oder verschiedene Substituenten aus der Gruppe Halogen, Nitro, Cyano, Trifluormethyl, Alkyl mit 1 bis 4 C-Atomen, Alkoxy mit 1 bis 4 C-Atomen besitzt,

$R^2$ den Rest eines 5-gliedrigen Rings mit mindestens einer Doppelbindung und mindestens 2 Heteroatomen oder Heteroatomgruppierungen aus der Reihe O, N, NH, S, wobei der 5-gliedrige Ring gegebenenfalls 1 oder 2 gleiche oder verschiedene Substituenten aus der Gruppe Alkyl mit 1 bis 4 C-Atomen, Alkylthio mit 1 bis 4 C-Atomen, Aralkyl mit insgesamt 7 bis 9 C-Atomen, Alkoxyalkyl mit insgesamt 2 bis 5 C-Atomen, Cycloalkyl mit 5 oder 6 C-Atomen, Aminocarbonylmethylthio, Methoxy-carbonyl, Ethoxy-carbonyl, Phenyl besitzt,

$R^4$ den Rest (R)-$CH(CH_3)CO_2R^5$, (S)-$CH(CH_3)CO_2R^5$ oder den Rest der Formel II

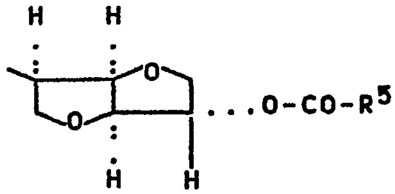 (II)

und

$R^5$ Alkyl mit 1 bis 6 C-Atomen

bedeuten, dadurch gekennzeichnet, daß

a) 1 mol einer Ylidenverbindung der Formel III

$$R^1-CH=\underset{|\\R^2}{C}-CO-CH_3 \quad (III)$$

und 1 mol eines 3-Aminocrotonsäureesters der Formel IV

$$H_3C-\underset{|\\NH_2}{C}=CH-CO_2R^4 \quad (IV)$$

oder daß

b) 1 mol eines Aldehyds der Formel V

$$R^1-CHO \quad (V),$$

1 mol eines 3-Aminocrotonsäureesters der Formel IV

$$H_3C-\underset{|\\NH_2}{C}=CH-CO_2R^4 \quad (IV)$$

und 1 mol eines Ketons der Formel VI

$$R^2-CH_2-CO-CH_3 \quad (VI)$$

oder daß

c) 1 mol einer Ylidenverbindung der Formel III

$$R^1-CH=\underset{|\\R^2}{C}-CO-CH_3 \quad (III),$$

1 mol eines Acetessigesters der Formel VII

$$H_3C-CO-CH_2-CO_2R^4 \quad (VII)$$

und 1 mol Ammoniak ($NH_3$)

oder daß

d) 1 mol einer Ylidenverbindung der Formel VIII

$$R^1\text{-CH=}\underset{\displaystyle CO_2R^4}{\underset{|}{C}}\text{-CO-CH}_3 \qquad \text{(VIII)}$$

und 1 mol eines Enamins der Formel IX

$$R^2\text{-CH=}\underset{\displaystyle NH_2}{\underset{|}{C}}\text{-CH}_3 \qquad \text{(IX)}$$

oder daß

e) 1 mol eines Aldehyds der Formel V

$$R^1\text{-CHO} \qquad \text{(V)},$$

1 mol eines Acetessigesters der Formel VII

$$H_3C\text{-CO-CH}_2\text{-CO}_2R^4 \qquad \text{(VII)}$$

1 mol eines Ketons der Formel VI

$$R^2\text{-CH}_2\text{-CO-CH}_3 \qquad \text{(VI)}$$

und 1 mol Ammoniak ($NH_3$)

oder daß

f) 1 mol eines Aldehyds der Formel V

$$R^1\text{-CHO} \qquad \text{(V)},$$

1 mol eines Acetessigesters der Formel VII

$$H_3C\text{-CO-CH}_2\text{-CO}_2R^4 \qquad \text{(VII)}$$

und 1 mol eines Enamins der Formel IX

$$R^2\text{-CH=}\underset{\displaystyle NH^2}{\underset{|}{C}}\text{-CO-CH}_3 \qquad \text{(IX)}$$

miteinander umgesetzt werden, wobei in den Verbindungen der Formel III bis IX $R^1$, $R^2$ und $R^4$ die eingangs genannten Bedeutungen besitzen, und aus dem bei der Umsetzung erhaltenen Gemisch von Diastereomeren eine optisch aktive Verbindung in an sich bekannter Weise isoliert und die so erhaltene optisch aktive Verbindung gegebenenfalls in an sich bekannter Weise in ein Säureadditionssalz überführt wird.

2. Verfahren nach Anspruch 1, dadurch gekennzeichnet, daß aus dem erhaltenen Gemisch von Diastereomeren der Formel Ia das als Hauptprodukt vorliegende optische Isomere durch Umkristallisation isoliert und gegebenenfalls in an

sich bekannter Weise in ein Säureadditionssalz überführt wird.

3. Verfahren zur Herstellung optisch aktiver, substituierter 1,4-Dihydropyridine der Formel Ib

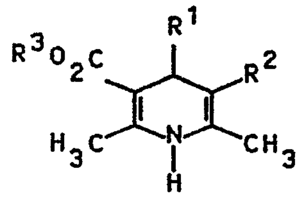

(Ib)

und ihrer Säureadditionsverbindungen, wobei in der Formel Ib

$R^1$ Pyridyl oder Thienyl, wobei der Pyridyl- oder Thienyl-Rest gegebenenfalls 1 oder 2 gleiche oder verschiedene Substituenten aus der Gruppe Alkyl mit 1 bis 4 C-Atomen, Alkoxy mit 1 bis 4 C-Atomen, Halogen, Trifluormethyl, Nitro, Cyano besitzt; Phenyl, das gegebenenfalls 1 oder 2 gleiche oder verschiedene Substituenten aus der Gruppe Halogen, Nitro, Cyano, Trifluormethyl, Alkyl mit 1 bis 4 C-Atomen, Alkoxy mit 1 bis 4 C-Atomen besitzt,

$R^2$ den Rest eines 5-gliedrigen Rings mit mindestens einer Doppelbindung und mindestens 2 Heteroatomen oder Heteroatomgruppierungen aus der Reihe O, N, NH, S, wobei der 5-gliedrige Ring gegebenenfalls 1 oder 2 gleiche oder verschiedene Substituenten aus der Gruppe Alkyl mit 1 bis 4 C-Atomen, Alkylthio mit 1 bis 4 C-Atomen, Aralkyl mit insgesamt 7 bis 9 C-Atomen, Alkoxyalkyl mit insgesamt 2 bis 5 C-Atomen, Cycloalkyl mit 5 oder 6 C-Atomen, Aminocarbonylmethylthio, Methoxy-carbonyl, Ethoxy-carbonyl, Phenyl besitzt,

$R^3$ Alkyl mit 1 bis 6 C-Atomen, Alkoxyalkyl mit 3 bis 8 C-Atomen, Dialkylaminoalkyl mit insgesamt 4 bis 9 C-Atomen, N-Aralkyl-N-Alkyl-aminoalkyl mit insgesamt 10 bis 14 C-Atomen, Cycloalkyl mit 5 oder 6 C-Atomen

bedeuten, dadurch gekennzeichnet, daß eine optisch aktive Verbindung der allgemeinen Formel Ia

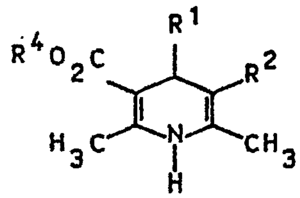

(Ia)

worin $R^1$ und $R^2$ die bereits angegebene Bedeutungen besitzen und

$R^4$ den Rest (R)-$CH(CH_3)CO_2R^5$, (S)-$CH(CH_3)CO_2R^5$ oder den Rest der Formel II

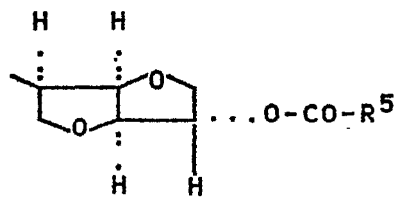

(II)

und

$R^5$ Alkyl mit 1 bis 6 C-Atomen

bedeuten, mit einem Alkohol der Formel $R^3OH$, wobei $R^3$ die angegebene Bedeutung besitzt, in an sich bekannter Weise umgeestert und die erhaltene Verbindung gegebenenfalls in an sich bekannter Weise in ein Säureadditionssalz überführt wird.

4. Verfahren nach Anspruch 3, dadurch gekennzeichnet, daß mit Isopropanol in Gegenwart von Lithium-, Natrium- oder Kalium-isopropanolat oder mit 2-Methoxyethanol in Gegenwart von Lithium-, Natrium-oder Kalium-2-methoxy-ethanolat umgeestert wird.

5. Optisch aktive, substituierte 1,4-Dihydropyridine der Formel I

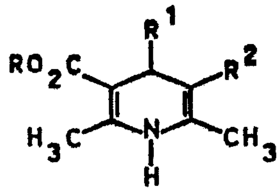

(I)

worin

R den Rest $R^3$ oder $R^4$,

$R^1$ Pyridyl oder Thienyl, wobei der Pyridyl- oder Thienyl-Rest gegebenenfalls 1 oder 2 gleiche oder verschiedene Substituenten aus der Gruppe Alkyl mit 1 bis 4 C-Atomen, Alkoxy mit 1 bis 4 C-Atomen, Halogen, Trifluormethyl, Nitro, Cyano besitzt; Phenyl, das gegebenenfalls 1 oder 2 gleiche oder verschiedene Substituenten aus der Gruppe Halogen, Nitro, Cyano, Trifluormethyl, Alkyl mit 1 bis 4 C-Atomen, Alkoxy mit 1 bis 4 C-Atomen besitzt,

$R^2$ den Rest eines 5-gliedrigen Rings mit mindestens einer Doppelbindung und mindestens 2 Heteroatomen oder Heteroatomgruppierungen aus der Reihe O, N, NH, S, wobei der 5-gliedrige Ring gegebenenfalls 1 oder 2 gleiche oder verschiedene Substituenten aus der Gruppe Alkyl mit 1 bis 4 C-Atomen, Alkylthio mit 1 bis 4 C-Atomen, Aralkyl mit insgesamt 7 bis 9 C-Atomen, Alkoxyalkyl mit insgesamt 2 bis 5 C-Atomen, Cycloalkyl mit 5 oder 6 C-Atomen, Aminocarbonylmethylthio, Methoxy-carbonyl, Ethoxy-carbonyl, Phenyl besitzt,

$R^3$ Alkyl mit 1 bis 6 C-Atomen, Alkoxyalkyl mit 3 bis 8 C-Atomen, Dialkylaminoalkyl mit insgesamt 4 bis 9 C-Atomen, N-Aralkyl-N-alkyl-aminoalkyl mit insgesamt 10 bis 14 C-Atomen, Cycloalkyl mit 5 bis 6 C-Atomen,

$R^4$ den Rest (R)-$CH(CH_3)CO_2R^5$, (S)-$CH(CH_3)CO_2R^5$ oder den Rest der Formel II

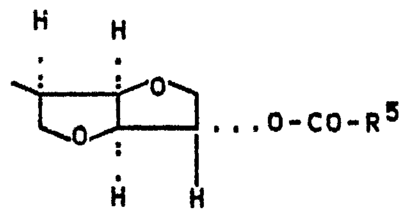 (II)

und

$R^5$ Alkyl mit 1 bis 6 C-Atomen

bedeuten, sowie ihre Säureadditionssalze.

6. Optisch aktive, substituierte 1,4-Dihydropyridine nach Anspruch 5, dadurch gekennzeichnet, daß R = $R^4$ und $R^5$ = Alkyl mit 1 bis 4 C Atomen bedeutet.

7. Optisch aktive, substituierte 1,4-Dihydropyridine nach Anspruch 5, dadurch gekennzeichnet, daß R = $R^3$ und $R^3$ Alkyl mit 1 bis 5 C-Atomen, insbesondere i-Propyl, Alkoxyalkyl mit 1 bis 4 C-Atomen im Alkoxyteil und 2 bis 4 C-Atomen im Alkylteil, insbesondere 2-Methoxy-ethyl, Dialkylaminoalkyl mit insgesamt 3 bis 6 C-Atomen, wobei jede der die Aminogruppe substituierenden Alkylgruppen 1 bis 3 C-Atome besitzt, bedeuten.

8. Optisch aktive, substituierte 1,4-Dihydropyridine nach einem oder mehreren der Ansprüche 5 bis 7, dadurch gekennzeichnet, daß $R^2$ für Oxadiazolyl oder ein durch Methyl, Ethyl, i-Propyl, tert.Butyl, Benzyl, Methylthio, i-Propylthio, Aminocarbonylthio, Methoxymethyl, substituiertes Oxadiazolyl steht.

9. 1,4-Dihydro-2,6-dimethyl-4-((2,3-dichlorphenyl)-5-(1,-3,4-oxadiazol-2-yl)-pyridin-3-carbonsäure-isopropylester in optisch aktiver Form, vorzugsweise in optisch aktiver linksdrehender Form.

10. 1,4-Dihydro-2,6-dimethyl-4-((2,3-dichlorphenyl)-5-(1,3,4-oxadiazol-2-yl)-pyridin-3-carbonsäure-(2-methoxy-ethyl)-ester in optisch aktiver Form.

11. Verwendung der optisch aktiven, insbesondere der optisch aktiven linksdrehenden, substituierten 1,4-Dihydropyridine der Ansprüche 5 bis 10 als Calciumantagonisten in pharmazeutischen Zubereitungen.

1

P A T E N T A N S P R Ü C H E für den Vertragsstaat Österreich

1. Verfahren zur Herstellung optisch aktiver, substituierter 1,4-Dihydropyridine der Formel Ia

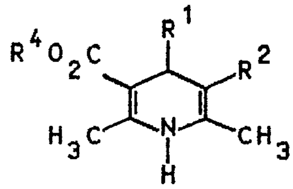

(Ia)

und ihrer Säureadditionssalze, wobei in der Formel Ia

$R^1$ Pidyl oder Thienyl, wobei der Pyridyl- oder Thienyl-Rest gegebenenfalls 1 oder 2 gleiche oder verschiedene Substituenten aus der Gruppe Alkyl mit 1 bis 4 C-Atomen, Alkoxy mit 1 bis 4 C-Atomen, Halogen, Trifluormethyl, Nitro, Cyano besitzt; Phenyl, das gegebenenfalls 1 oder 2 gleiche oder verschiedene Substituenten aus der Gruppe Halogen, Nitro, Cyano, Trifluormethyl, Alkyl mit 1 bis 4 C-Atomen, Alkoxy mit 1 bis 4 C-Atomen besitzt,

$R^2$ den Rest eines 5-gliedrigen Rings mit mindestens einer Doppelbindung und mindestens 2 Heteroatomen oder Heteroatomgruppierungen aus der Reihe O, N, NH, S, wobei der 5-gliedrige Ring gegebenenfalls 1 oder 2 gleiche oder verschiedene Substituenten aus der Gruppe Alkyl mit 1 bis 4 C-Atomen, Alkylthio mit 1 bis 4 C-Atomen, Aralkyl mit insgesamt 7 bis 9 C-Atomen, Alkoxyalkyl mit insgesamt 2 bis 5 C-Atomen, Cycloalkyl mit 5 oder 6 C-Atomen, Aminocarbonylmethylthio, Methoxy-carbonyl, Ethoxy-carbonyl, Phenyl besitzt,

$R^4$ den Rest (R)-$CH(CH_3)CO_2R^5$, (S)-$CH(CH_3)CO_2R^5$ oder den Rest der Formel II

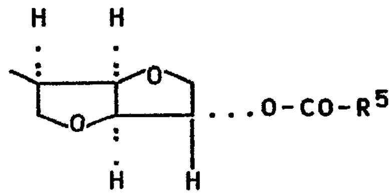 (II)

und

$R^5$ Alkyl mit 1 bis 6 C-Atomen

bedeuten, dadurch gekennzeichnet, daß

a) 1 mol einer Ylidenverbindung der Formel III

$$R^1-CH=C(R^2)-CO-CH_3 \quad (III)$$

und 1 mol eines 3-Aminocrotonsäureesters der Formel IV

$$H_3C-C(NH_2)=CH-CO_2R^4 \quad (IV)$$

oder daß

b) 1 mol eines Aldehyds der Formel V

$$R^1-CHO \quad (V),$$

1 mol eines 3-Aminocrotonsäureesters der Formel IV

$$H_3C-C(NH_2)=CH-CO_2R^4 \quad (IV)$$

und 1 mol eines Ketons der Formel VI

$$R^2-CH_2-CO-CH_3 \quad (VI)$$

oder daß

c) 1 mol einer Ylidenverbindung der Formel III

$$R^1-CH=C(R^2)-CO-CH_3 \quad (III),$$

1 mol eines Acetessigesters der Formel VII

$$H_3C-CO-CH_2-CO_2R^4 \quad (VII)$$

und 1 mol Ammoniak ($NH_3$)

oder daß

d) 1 mol einer Ylidenverbindung der Formel VIII

$R^1$-CH=C-CO-$CH_3$ (VIII)
|
$CO_2R^4$

und 1 mol eines Enamins der Formel IX

$R^2$-CH=C-$CH_3$ (IX)
|
$NH_2$

oder daß

e) 1 mol eines Aldehyds der Formel V

$R^1$-CHO (V),

1 mol eines Acetessigesters der Formel VII

$H_3C$-CO-$CH_2$-$CO_2R^4$ (VII)

1 mol eines Ketons der Formel VI

$R^2$-$CH_2$-CO-$CH_3$ (VI)

und 1 mol Ammoniak ($NH_3$)

oder daß

f) 1 mol eines Aldehyds der Formel V

$R^1$-CHO (V),

1 mol eines Acetessigesters der Formel VII

$H_3C$-CO-$CH_2$-$CO_2R^4$ (VII)

und 1 mol eines Enamins der Formel IX

$R^2$-CH=C-CO-$CH_3$ (IX)
|
$NH^2$

miteinander umgesetzt werden, wobei in den Verbindungen der Formel III bis IX $R^1$, $R^2$ und $R^4$ die eingangs genannten Bedeutungen besitzen, und aus dem bei der Umsetzung erhaltenen Gemisch von Diastereomeren eine optisch aktive Verbindung in an sich bekannter Weise isoliert und die so erhaltene optisch aktive Verbindung gegebenenfalls in an sich bekannter Weise in ein Säureadditionssalz überführt wird.

2. Verfahren nach Anspruch 1, dadurch gekennzeichnet, daß aus dem erhaltenen Gemisch von Diastereomeren der Formel Ia das als Hauptprodukt vorliegende optische Isomere durch Umkristallisation isoliert und gegebenenfalls in an

sich bekannter Weise in ein Säureadditionssalz überführt wird.

3. Verfahren zur Herstellung optisch aktiver, substituierter 1,4-Dihydropyridine der Formel Ib

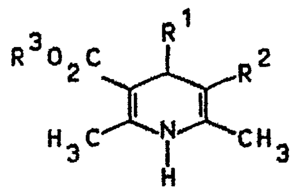

(Ib)

und ihrer Säureadditionsverbindungen, wobei in der Formel Ib

$R^1$ Pyridyl oder Thienyl, wobei der Pyridyl- oder Thienyl-Rest gegebenenfalls 1 oder 2 gleiche oder verschiedene Substituenten aus der Gruppe Alkyl mit 1 bis 4 C-Atomen, Alkoxy mit 1 bis 4 C-Atomen, Halogen, Trifluormethyl, Nitro, Cyano besitzt; Phenyl, das gegebenenfalls 1 oder 2 gleiche oder verschiedene Substituenten aus der Gruppe Halogen, Nitro, Cyano, Trifluormethyl, Alkyl mit 1 bis 4 C-Atomen, Alkoxy mit 1 bis 4 C-Atomen besitzt,

$R^2$ den Rest eines 5-gliedrigen Rings mit mindestens einer Doppelbindung und mindestens 2 Heteroatomen oder Heteroatomgruppierungen aus der Reihe O, N, NH, S, wobei der 5-gliedrige Ring gegebenenfalls 1 oder 2 gleiche oder verschiedene Substituenten aus der Gruppe Alkyl mit 1 bis 4 C-Atomen, Alkylthio mit 1 bis 4 C-Atomen, Aralkyl mit insgesamt 7 bis 9 C-Atomen, Alkoxyalkyl mit insgesamt 2 bis 5 C-Atomen, Cycloalkyl mit 5 oder 6 C-Atomen, Aminocarbonylmethylthio, Methoxy-carbonyl, Ethoxy-carbonyl, Phenyl besitzt,

$R^3$ Alkyl mit 1 bis 6 C-Atomen, Alkoxyalkyl mit 3 bis 8 C-Atomen, Dialkylaminoalkyl mit insgesamt 4 bis 9 C-Atomen, N-Aralkyl-N-Alkyl-aminoalkyl mit insgesamt 10 bis 14 C-Atomen, Cycloalkyl mit 5 oder 6 C-Atomen

bedeuten, dadurch gekennzeichnet, daß eine optisch aktive Verbindung der allgemeinen Formel Ia

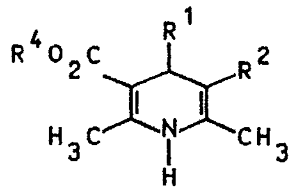

(Ia)

worin $R^1$ und $R^2$ die bereits angegebene Bedeutungen besitzen und

$R^4$ den Rest (R)-$CH(CH_3)CO_2R^5$, (S)-$CH(CH_3)CO_2R^5$ oder den Rest der Formel II

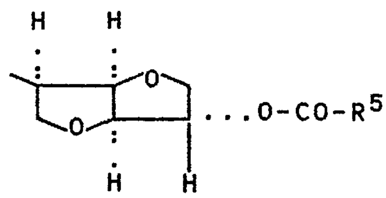

(II)

und

$R^5$ Alkyl mit 1 bis 6 C-Atomen

bedeuten, mit einem Alkohol der Formel $R^3OH$, wobei $R^3$ die angegebene Bedeutung besitzt, in an sich bekannter Weise umgeestert und die erhaltene Verbindung gegebenenfalls in an sich bekannter Weise in ein Säureadditionssalz überführt wird.

4. Verfahren nach Anspruch 3, dadurch gekennzeichnet, daß mit Isopropanol in Gegenwart von Lithium-, Natrium- oder Kalium-isopropanolat oder mit 2-Methoxyethanol in Gegenwart von Lithium-, Natrium-oder Kalium-2-methoxy-ethanolat umgeestert wird.

5. Verfahren nach Anspruch 1, dadurch gekennzeichnet, daß die Ausgangsverbindungen so ausgewählt werden, daß Verbindungen der Formel Ia entstehen, wobei $R^5$ = Alkyl mit 1 bis 4 C Atomen bedeutet.

6. Verfahren nach Anspruch 3, dadurch gekennzeichnet, daß die Ausgangsverbindungen so gewählt werden, daß Verbindungen der Formel Ib entstehen, wobei $R^3$ Alkyl mit 1 bis 5 C-Atomen, insbesondere i-Propyl, Alkoxyalkyl mit 1 bis 4 C-Atomen im Alkoxyteil und 2 bis 4 C-Atomen im Alkylteil, insbesondere 2-Methoxy-ethyl, Dialkylaminoalkyl mit insgesamt 3 bis 6 C-Atomen, wobei jede der die Aminogruppe substituierenden Alkylgruppen 1 bis 3 C-Atome besitzt, bedeutet.

7. Verfahren nach einem oder mehreren der Ansprüche 1 bis 6, dadurch gekennzeichnet, daß die Ausgangsverbindungen so ausgewählt werden, daß Verbindungen der Formel Ia oder Ib entstehen, wobei $R^2$ für Oxadiazolyl oder ein durch Methyl, Ethyl, i-Propyl, tert.Butyl, Benzyl, Methylthio, i-Propylthio, Aminocarbonylthio, Methoxymethyl, substituiertes Oxadiazolyl steht.

8. Verfahren nach Anspruch 3 und/oder 4, dadurch gekennzeichnet, daß die Ausgangsverbindungen so ausgewählt werden, daß 1,4-Dihydro-2,6-dimethyl-4-((2,3-dichlorphenyl)-5-(1,3,4-oxadiazol-2-yl)-pyridin-3-carbonsäure-isopropylester in optisch aktiver Form, vorzugsweise in optisch aktiver linksdrehender Form, entsteht.

9. Verfahren nach Anspruch 3 und/oder 4, dadurch gekennzeichnet, daß die Ausgangsverbindungen so ausgewählt werden, daß 1,4-Dihydro-2,6-dimethyl-4-((2,3-dichlorphenyl)-5-(1,3,4-oxadiazol-2-yl)-pyridin-3-carbonsäure-(2-methoxy-ethyl)-ester in optisch aktiver Form entsteht.

10. Verfahren zur Herstellung eines calcium-antagonistisch wirksamen Mittels, dadurch gekennzeichnet, daß man optisch aktives, substituiertes 1,4-Dihydropyridin der

Formel I

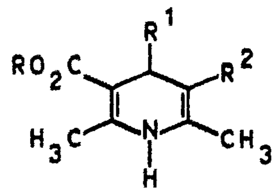

(I)

worin

R den Rest $R^3$ oder $R^4$,

$R^1$ Pyridyl oder Thienyl, wobei der Pyridyl- oder Thienyl-Rest gegebenenfalls 1 oder 2 gleiche oder verschiedene Substituenten aus der Gruppe Alkyl mit 1 bis 4 C-Atomen, Alkoxy mit 1 bis 4 C-Atomen, Halogen, Trifluormethyl, Nitro, Cyano besitzt; Phenyl, das gegebenenfalls 1 oder 2 gleiche oder verschiedene Substituenten aus der Gruppe Halogen, Nitro, Cyano, Trifluormethyl, Alkyl mit 1 bis 4 C-Atomen, Alkoxy mit 1 bis 4 C-Atomen besitzt,

$R^2$ den Rest eines 5-gliedrigen Rings mit mindestens einer Doppelbindung und mindestens 2 Heteroatomen oder Heteroatomgruppierungen aus der Reihe O, N, NH, S, wobei der 5-gliedrige Ring gegebenenfalls 1 oder 2 gleiche oder verschiedene Substituenten aus der Gruppe Alkyl mit 1 bis 4 C-Atomen, Alkylthio mit 1 bis 4 C-Atomen, Aralkyl mit insgesamt 7 bis 9 C-Atomen, Alkoxyalkyl mit insgesamt 2 bis 5 C-Atomen, Cycloalkyl mit 5 oder 6 C-Atomen, Aminocarbonylmethylthio, Methoxy-carbonyl, Ethoxy-carbonyl, Phenyl besitzt,

$R^3$ Alkyl mit 1 bis 6 C-Atomen, Alkoxyalkyl mit 3 bis 8 C-Atomen, Dialkylaminoalkyl mit insgesamt 4 bis 9 C-Atomen, N-Aralkyl-N-alkyl-aminoalkyl mit insgesamt 10 bis 14 C-Atomen, Cycloalkyl mit 5 bis 6 C-Atomen,

$R^4$ den Rest (R)-$CH(CH_3)CO_2R^5$, (S)-$CH(CH_3)CO_2R^5$ oder den Rest der Formel II

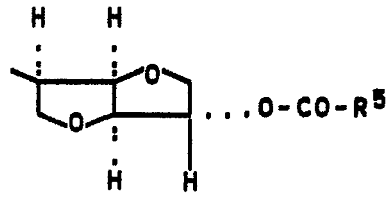

(II)

und

$R^5$ Alkyl mit 1 bis 6 C-Atomen

bedeuten, oder ein Säureadditionssalz davon nach einem Verfahren der Ansprüche 1 bis 9 herstellt und daß man die so erhaltene Verbindung der Formel I oder ein Säureadditionssalz davon mit einem geeigneten pharmazeutisch annehmbaren Trägerstoff mischt.